# EUROPÄISCHE PATENTSCHRIFT

(11) **EP 2 515 757 B1**
(45) Veröffentlichungstag und Bekanntmachung des Hinweises auf die Patenterteilung: **12.03.2014**
(21) Anmeldenummer: 10703001.7
(22) Anmeldetag: 27.01.2010
(51) Int. Cl.: A61B 5/107, G01B 11/25

(54) **KALIBRATIONSFREIE UND GENAUE OPTISCHE ERFASSUNG DER RAUMFORM**
CALIBRATION-FREE AND PRECISE OPTICAL DETECTION OF A THREE-DIMENSIONAL SHAPE
DÉTECTION OPTIQUE PRÉCISE ET SANS ÉTALONNAGE DE LA FORME SPATIALE

(30) Priorität: 22.12.2009 DE 202009017401 U
(43) Veröffentlichungstag der Anmeldung: 31.10.2012
(73) Patentinhaber: Corpus.E AG, 70178 Stuttgart (DE)
(72) Erfinder: PFEIFFER, Rene, 71706 Markgröningen (DE); RUTSCHMANN, Dirk, 70180 Stuttgart (DE)
(74) Vertreter: Prinz & Partner
(86) Internationale Anmeldenummer: PCT/EP2010/000479
(87) Internationale Veröffentlichungsnummer: WO 2011/076292

(56) Entgegenhaltungen:
- WO-A1-2009/065418
- DE-A1- 2 032 282
- DE-A1- 10 156 908
- US-A1- 2004 234 122

## Beschreibung

Die Erfindung betrifft ein Verfahren und ein Gerät zur kalibrationsfreien, kostengünstigen und genauen optischen Erfassung der Raumform von Körpern und Körperteilen.

Die Erfassung der 3-dimensionalen Raumform von Körpern und Körperteilen, insbesondere von menschlichen Körperteilen wie Beine, Rumpf und Füße, ist ein wichtiger Bestandteil bei der Herstellung oder Zuordnung passender Bekleidungsstücke, orthopädischer Hilfsmittel wie Kompressionsstrümpfe, Prothesen und Orthesen als auch bei der Herstellung bzw. Zuordnung passender Schuhe. Solche Körperscanner (engl. body scanner) werden darüber hinaus auch immer häufiger in Form von Ganzkörperscannern im Bereich der medizinischen Diagnose von orthopädischen Befunden wie Rückenskoliose, des Therapie-Monitoring von Lymphödemen und von kosmetisch-chirurgischen Eingriffen wie Fettabsaugung usw. genutzt.

Alle diese Anwendungen verlangen nach "echten" 3D Scannern, d.h. Scanner welche in der Lage sind, ein vollständiges 3D Modell sowohl der konvexen als auch der konkaven Teile des Körpers zu erfassen. Es werden daher bei der Betrachtung des Standes der Technik pseudo-3D Verfahren wie das Silhouetten-Verfahren (Aufnahme der Silhouette des Körpers aus mehreren Richtungen) ausgeschlossen, da diese Verfahren in der Regel kein vollständiges 3D Modell liefern können.

Von den Anwendern von Scannern wird aus Kostengründen zurecht erwartet, dass unterschiedliche Einsatzgebiete und Aufgaben soweit als möglich mit dem gleichen Body-Scanner bedient werden können und die für den speziellen Einsatz benötigten Raum-Daten als Untermenge aus dem erstellten vollständigen 3D Datensatz des gesamten Körpers entnommen werden. Nur so lässt sich eine unwirtschaftliche Aufsplitterung der gestellten Aufgaben auf eine große Zahl spezifischer Scanner wie Fußscanner, Rückenscanner, Rumpfscanner, Kniescanner, Schulterscanner usw. vermeiden.

Dieser Wunsch nach einem Ganzkörperscanner stellt hohe Anforderungen an den von diesen Scannern abzudeckenden Messraum (ca. 1 qm Grundfläche x 2 m Höhe), der erforderlichen hohen Auflösung von typ. 1000 Raumpunkte pro Kubikzentimeter und der hohen und vor allem langfristig konstanten Genauigkeit des erstellten Raummodells von ca. 0,5 mm.

Neben diesen eher technischen Spezifikationen müssen diese Ganzkörper-Scanner eine weitere Reihe von Bedingungen erfüllen, um in den sehr kostenkritischen Bereichen der Medizin, der para- und Sanitär-medizinischen Industrie sowie des "mass-customization" von Bekleidung und Schutzkleidung wirtschaftlich erfolgreich eingesetzt zu werden:
a) der Körperscanner muss mit einer möglichst geringen Stellfläche auskommen, weil der zur Verfügung stehende Raum oft sehr eingeschränkt ist;
b) der Körperscanner muss bei normalen Umgebungslichtverhältnissen arbeiten können, d.h. ohne den Einbau in dunkel abgeschottete Kabinen, da bei zahlreichen Kunden hierdurch Ängste und Abneigung entstehen.
c) Der Körperscanner muss zuverlässig auch von nur wenig ausgebildetem Personal bedienbar sein. Dies bedingt zwingend die Vermeidung von häufigen und aufwändigen Re-Kalibrierungsverfahren, welche ein solches Personal überfordern.
d) Die Herstellkosten und die Betriebskosten müssen trotz dieser hohen Anforderungen sehr niedrig liegen, um in den genannten kosten-sensitiven Geschäftsbereichen eingesetzt werden zu können.

Es sind mittlerweile zahlreiche optische 3D Körper-Scanner auf dem Markt, welche meistens entweder mit den Verfahren des Laser-Lichtschnitts (siehe z.B. Firma Vitronic Dr. Stein, www. http://www.vitronic.de/bodyscannen) oder der Streifenprojektion (siehe z.B. bodyScan der Firma Breuckmann, www.breuckmann.com) arbeiten. Beide Verfahren beruhen auf der Grundlage der Triangulation, d.h. einer sehr stabilen und genauen räumlichen Dreiecksanordnung von Lichtprojektor, Kamera und Körper zur punktweise Bestimmung der Entfernung der beobachten Körperfläche von dem Kamera-Projektor-Meßkopf. Aus der Summe dieser Entfernungsdaten wird ein XYZ Punktemodell der betrachten Körperoberfläche in einem Welt- und/oder Objektbezogenen Koordinatensystem erstellt. In weiteren Verarbeitungsschritten wird hieraus zumeist durch Dreiecks-Vermaschung ein für die weitere Verarbeitung geeignetes 3D Modell in einem der zahlreichen standardisierten Formate wie .dxf, VRML,STEP etc. berechnet.

Um den gesamten Körper zu erfassen, müssen entweder mehrere Kamera/Projektor-Anordnungen um den Körper herum angebracht werden (z.B. beim bodyScan der Firma Breuckmann), oder es müssen mehrere Kamera/Projektor-Anordnungen mechanisch über die Köperoberfläche bewegt werden (z.B. beim VITUS Ganzkörperscanner der Firma Vitronic Dr. Stein).

Eine ausführliche Darstellung der derzeitig kommerziell verfügbaren Körperscanner und der eingesetzten Technologien ist über das Internet-Portal www.hometrica.ch zu finden.

Die erforderliche Winkelanordnung von Kamera zu Projektor ist empfindlich: bereits kleine Winkelfehler führen zu großen Messfehlern in den gemessenen Entfernungen. Die Bewegung der Kamera/Projektor-Anordnung im Raum ist gleichermaßen empfindlich: kleine Fehler bei der Positionsbestimmung des Messkopfes während einer Aufnahme führen zu großen Messfehlern im erzeugten 3D Modell. Diese Empfindlichkeit führt dazu, dass alle auf der Triangulation aufbauenden Scanner sogar bei einer sehr robusten und aufwendigen opto-mechanischen Bauweise trotzdem häufig neukalibriert werden müssen, insbesondere auch nach jedem Transport und bei jedem Verschieben des Scanners.

Die erforderliche Re-Kalibrierung eines auf der Triangulation mit der Laser-oder Streifenprojektion arbeitenden 3D Scanners benötigt verschiedene Normalen wie Normkörper, markierte Platten usw. und erzeugt eine große Zahl von Parametern, welche für die Gewährleistung der Messgenauigkeit erforderlich sind. Dies sind z.B. :
- die genaue Raumlage zwischen Kamera und Projektor (Triangulationswinkel, Messbasis, gegenseitige Ausrichtung, ...)
- die genauen inneren Parameter von Kamera und Projektor (Brennweiten, Sensordimension, Geometrie der Bildpunkte, Kipp- und Drehwinkel des Laserlinienprojektors ...)
- die genauen Raumpositionen des Kamera/Projektor-Messkopfes bei jeder Messaufnahme , die sog. äußeren Parameter
- etc.

Daher ist die Re-Kalibrierung eines 3D Scanners in der Regel ein aufwendiger Vorgang, bei welchem das Kaufpersonal z.B. eines orthopädischen Fachgeschäftes häufig überfordert ist und damit auch geneigt ist, diese Technologie abzulehnen.

Die heutigen auf der Triangulation aufbauenden 3D Ganzkörper-Scanner können wegen der benötigten mechanischen Stabilität auch nicht sehr preiswert angeboten werden, so dass derzeit viele potentielle Anwendungen wegen der hohen Kosten der 3D Scanner nicht kommerziell umgesetzt werden.

Die Firma corpus.e AG (www.corpus-e.com) hat unter der Bezeichnung "lightbeam®" einen photogrammetrischen Fußscanner entwickelt, welcher ohne Projektor und ohne empfindliche Triangulations-Anordnung arbeitet (siehe www.corpus-e.com). Hierbei wird der Fuß mit einem speziell photogrammetrisch markiertem elastischen Socken bekleidet und eine Videokamera mechanisch um den Fuß herum bewegt (siehe WO 2004/078040 A1).

Der Fuß steht auf einer photogrammetrisch markierten Unterlage, so dass die Raumposition, aus welcher die Kamera misst, permanent automatisch mit den Verfahren der Photogrammetrie bestimmt werden kann (die sog." äusseren" Parameter einer photogrammetrischen Messanordnung). Ebenso können die sog. "inneren" Parameter der Kamera selbst wie Brennweite, Bildsensor, Durchstoßpunkt der optischen Achse, Objektiwerzerrungen usw.) automatisch aus der Auswertung überlappender 2D Aufnahmen der markierten Unterlage und des markierten Fußes bestimmt werden. Damit ist dieses System völlig kalibrierungsfrei bzw. genauer ausgedrückt, inhärent selbst-kalibrierend. Es kann jederzeit nach einem Transport ohne Kalibrierung durch den Anwender in Betrieb genommen werden; es braucht niemals nach einem Lastwechsel nachkalibriert werden; die mechanische Stabilität der Konstruktion kann einfach und preiswert sein, da sie nicht in das Endergebnis, das gemessene 3D Modell, einfließt.

Es gibt allerdings einen Nachteil dieses ansonsten mächtigen Verfahrens: durch die textilbedingte beschränkte Dichte der photogrammetrischen Markierungen auf dem elastischen Socken ist die Dichte der erzeugten XYZ Punktewolke gegenüber einem Laser- oder Streifenprojektionsverfahren deutlich niedriger (typ. 4000 XYZ Punkte gegenüber ca. 1 Million XYZ Punkte bei einem Lichtschnitt-Scanner). Während diese geringere Punktedichte bei flachen Körperteilen wie dem Oberfuß keinen Nachteil darstellt, kann sie in dem Bereich hoher Raumkrümmungen wie bei den Zehen, der Ferse, beim Übergang vom Oberfuß zur Fußsohle usw. einschränkend sein.

Auch die DE 101 56 908 A1 offenbart ein Verfahren zur Erfassung der Raumform von Körpern, bei dem der zu vermessende Körper komplett mit einem fotogrammetrisch markierten elastischen Überzug bekleidet wird. Der Körper wird auf eine Platte gestellt, die fotogrammetrisch auswertbare Marken aufweist. Überlappende Bildaufnahmen werden von einem Operator freihändig gemacht.

Die Forderung, dass der zu vermessende Körper komplett mit einem photogrammetrisch markierten elastischen Überzug bekleidet werden muss, stellt einen weiteren Nachteil dar. Solche Überzüge sind nicht einfach zu produzieren; es werden mehrere Größen und Formen je nach Körperbau des Kunden und je nach interessiertem Körperteil wie Rumpf, Beine, Fuß, Schulter, Hände usw. benötigt.

Es gibt auch Scan-Anwendungen wie z.B. das Digitalisieren von Füßen für die Auswahl geeigneter Ski-Stiefel, bei welchen es wichtig ist, dass der Kunde seine eigenen Wintersocken anbehält, damit diese bei der Formanpassung mit berücksichtigt werden. Es ist aber nicht möglich, mit einfachen Mitteln einen beliebigen Socken nachträglich photogrammetrisch zu markieren.

Damit ist trotz der erreichten Kalibrationsfreiheit dieses System nicht besonders für einen kostengünstigen Ganzkörperscanner geeignet.

Unter der DE196536294 A1 wird von Malz et al. ein allgemeines Verfahren der 3D Digitalisierung von Gegenständen mit Hilfe einer Kamera/Lichtprojektor-Triangulations-Anordnung beschrieben, bei welchem der gesamte Messraum mit photogrammetrisch markierten Seitenwänden versehen ist und darüber hinaus weitere photogrammetrisch markierte Kalibrationskörper in den Messraum gestellt werden. Dieses Verfahren ermöglicht die explizite in-situ Kalibrierung der Messanordnung.

Diese Methodik ist im Vermessungslabor einsetzbar, wo gut ausgebildetes Personal vor allem starre mechanischen Modelle digitalisiert, wie z.B. bei der Automobil-Entwicklung. Sie ist allerdings wenig geeignet für einen kostengünstigen Ganzkörperscanner, bei welchem die Anzahl der photogrammetrisch markierten Flächen in der Messkabine eines Sanitäts-Fachgeschäftes klein und möglichst auch ästhetisch nicht störend sein soll, wo keinerlei manuell aufzustellende Kalibrationskörper oder -paneele benötigt werden sollen und insgesamt der Eindruck eines geschlossenen Messraumes vermieden werden soll. Diese von Malz beschriebene Methode ist auch wenig für einen Ganzkörperscanner geeignet, mit welchem der menschliche Körper rundherum, d.h. vollständig aus einer Vielzahl von Ansichten möglichst schnell digitalisiert werden soll.

In der WO 2009/065418 A1 vom 28.5.2009 wird von Rutschmann und Pfeiffer ein Körperscanner vorgestellt, welcher ebenfalls ohne photogrammetrisch markierte textile Überzüge auskommt und trotzdem das angestrebte Prinzip der Kalibrationsfreiheit (bzw. der impliziten Re-Kalibration) beibehält. Dieses Verfahren verwendet eine photogrammetrisch markierte Grundplatte, auf welcher der Kunde steht, und eine mechanisch auf einer ungefähren Kreisbahn um den Körper herum bewegte Laserlinien/Kamera-Triangulationsanordnung. Hierbei ist die Kamera so ausgerichtet, daß sie bei jeder Position auf der Umlaufbahn einen Ausschnitt der Bodenmarkierungen mit erfaßt sowie in einer höheren Position etwa in der Bildmitte ein elastisches, photogrammetrisch markiertes Band, welches z.B. um das Bein des Kunden geschlungen ist, ebenfalls miterfaßt. Die Kamera erfaßt damit gleichzeitig bei jeder Aufnahme folgende Elemente:
a) die (mindestens eine) helle Lichtlinie des Laserprojektors auf der Körperoberfläche, welche vertikal durch das Bildfeld verläuft,
b) die gleiche Linie im unteren Teil des Bildfeldes, welche eine helle Spur auf der photogrammetrischen Grundplatte erzeugt,
c) die helle Spur der Lichtlinie auf dem elastischen Band ungefähr in Bildmitte.

Unter der einzigen konstruktiven Voraussetzung, dass die erzeugte Laserlinie geradlinig ist, können aus den zahlreichen Kameraaufnahmen um den Körper herum sowohl die inneren Parameter der Kamera als auch die äußeren Parameter des Linienprojektor/Kamera-Messkopfes (Triangulationswinkel, Messbasis, Orientierung im Raum) mit bekannten photogrammetrischen Verfahren bestimmt werden. Damit ist dieses System inhärent selbstkalibrierend; jede mechanische Veränderung der kritischen Komponenten Linienprojektor und Kamera, d.h. sämtliche inneren und äußeren Parameter werden bei jeder Digitalisierung photogrammetrisch mitbestimmt.

Für den Benutzer ergibt sich hieraus ein bedeutender Vorteil: diese Scanner brauchen nicht vom Benutzer re-kalibriert zu werden. Für den Produzenten bedeutet diese inhärente Selbstkalibrierung, daß keine extrem steifen und genauen Konstruktionen, keine aufwendige Justagen usw. erforderlich sind und damit solche Körperscanner kostengünstig zu produzieren sind und einfach beim Kunden in Betrieb genommen werden können.

Allerdings hat das von Rutschmann et al. beschriebene Verfahren bei allen Vorteilen doch noch eine Reihe von Nachteilen:
a) Durch die Bewegung der Kamera/Linienprojektor-Anordnung um den Körper des Kunden herum wird ein relativ großer zylinderförmiger Raum benötigt; ein solcher Scanner benötigt typischerweise eine kreisförmigen Grundfläche von etwa 3 m Durchmesser, da Mindestabstände zwischen Kamera/Laser-Messkopf und Körper des Kunden eingehalten werden müssen. Viele Sanitätshäuser und Kliniken verfügen nicht über solche großen Räume, sondern erwarten einen Ganzkörperscanner, welcher möglichst wenig Stellfläche benötigt.
b) Das manuelle Anbringen eines photogrammetrisch markierten Bandes oder einer ähnlichen Marke im Bildfeld ist eine Fehlerquelle und setzt ein gewisses Maß an optischem Verständnis voraus, welches beim Verkaufspersonal oft nicht vorhanden ist.
c) Außerdem ist bei der WO 2009/065418 A1 das Problem nicht gelöst, einen Ganzkörperscanner zu realisieren, welcher zumindest von den Füssen bis zur Schulter/Halspartie reicht. Die aufgezeigte Kamera/Linienprojektor-Anordnung ist auch bei extremen Weitwinkel-Kameras nicht in der Lage, die obere Fläche der Schulter mit dem Linienprojektor zu beleuchten und optisch gleichzeitig mit der photogrammetrisch markierten Bodenplatte zu erfassen, oder aber die okkludierten Teile des Unterbauches bildhaft zu erfassen.
d) WO 2009/065418 A1 gibt auch keine Lehre, wie zwischen der Vielfalt der photogrammetrischen Marken die Lichtspur des Linienprojektors deutlich zu erkennen und aus dem Kamerabild heraus auszuwerten ist.
e) Abweichungen von der konstruktionsbedingten Geradlinigkeit des Linienprojektors werden nicht erkannt und können nicht automatisch korrigiert werden.

Es besteht daher ein wirtschaftliches und technisches Interesse an einem kostengünstigen, einfach zu bedienenden und kalibrationsfreien bzw. inhärent rekalibrierten Körper-Scanner zur hochauflösenden Erfassung der anatomischen Raumform von vorwiegend menschlichen Körpern, welcher kein photogrammetrisch markiertes Messtextil benötigt, mit einem kleinvolumigen Messaufbau auskommt und ein so genaues und dichtes 3D Modell automatisch erzeugt, dass es für eine Vielzahl von Anwendungen vom medizinischen und orthopädischen Bereich bis zum mass-customization von Fuß- und Körperbekleidung eingesetzt werden kann.

Dieses Ziel wird mit dem in den Patentansprüchen angegebenen Verfahren und mit einer in den Patentansprüchen angegebenen Erfassungsanordnung erreicht.

Kurz umrissen stellt sich die Erfindung wie folgt dar. Der Kunde steht auf einem motorisch oder per Hand bewegten Drehteller. Mindestens eine Lichtlinie, die im wesentlichen senkrecht zur Ebene des Drehtellers ausgerichtet ist, wird von einem Triangulationsmesskopf auf den Kunden projiziert. Der ebene Drehteller ist außerhalb des Fußbereiches mit codierten, photogrammetrisch auswertbaren Marken versehen, welche durch mindestens ein markierungsfreies Feld unterbrochen sind. Eine weitere, mit codierten, photogrammetrisch auswertbaren Marken versehene Fläche befindet sich im wesentlichen senkrecht oberhalb der Drehplatte und vorzugsweise an Pfosten oder Haltestegen, an denen sich der Kunde bei der Drehung des Drehtellers festhält. Auch diese markierte Fläche ist durch mindestens ein markierungsfreies Feld unterbrochen. Während der Drehbewegung des Körpers mit dem Drehteller werden alle sichtbaren photogrammetrischen Marken in den jeweils von der Kamera erfassten Bildern aufgenommen, ebenso wie die darin eingelagerten markierungsfreien Felder, und die entsprechenden Signale der Kamera werden im Triangulationsmesskopf fortlaufend in einen Rechner übertragen. Die Lage der markierungsfreien Feldern relativ zu den umgebenden oder angrenzenden photogrammetrischen Marken ist dem Rechner bekannt, z.B. in Form einer Liste oder Tabelle der Marken, welche Begrenzungsmarken für die markierungsfreien Felder sind. Mit einem photogrammetrischen Programm wird in allen Bildern die von dem mindestens einen Lichtprojektor des Triangulationsmesskopfes auf den zu digitalisierenden Körper projizierte Lichtspur zur Bestimmung des 3D Modells des Körpers nach bekannten Verfahren der Triangulation ausgewertet. Gleichzeitig wird mit Hilfe eines photogrammetrischen Programms aus einer Vielzahl dieser Bilder die Lage der photogrammetrischen Marken auf dem Drehteller und auf den Pfosten oder Haltestegen, an denen sich der Kunde während der Drehung festhält, ausgewertet. Ferner wird die Lage der linienhaften Lichtspuren des mindestens einen Linienprojektors in den markierungsfreien Feldern des Drehtellers und des Haltesteges bestimmt. Aus beiden Auswertungen werden mit Hilfe des genannten photogrammetrischen Programs alle inneren und äußeren Parameter des mindestens einen Triangulationsmeßkopfes bestimmt und zur inhärenten Selbstkalibrierung des 3D Modells verwendet. Der Verlauf der Lichtspur auf einem unmarkierten Feld des Drehtellers ermöglicht nämlich die Ableitung des Triangulationswinkels zwischen Kamera und projizierter Lichtebene in der XY-Ebene, d.h. in Bezug auf die Grundfläche des Weltkoordinatensystems XYZ. Der Verlauf der Lichtspur auf einem unmarkierten Feld der Haltestege ermöglicht andererseits die Bestimmung der Verkantung des Linienprojektors, d.h. des Drehwinkels in der ZX-Ebene. Allerdings muss hierfür die Lage der unmarkierten Fläche des Haltsteges im Raum bekannt sein. Die Lage dieser nicht-markierten Fläche(n) im Raum wird mit Hilfe der absoluten Markierungen der Pfosten oder Haltestege photogrammetrisch aus den Kamerabildern während der Drehbewegung ermittelt. Auch Abweichungen in der Geradlinigkeit der projizierten Linie, beispielsweise durch Fehler oder Dejustagen der Strahlformungsoptiken des Linienprojektors, können kalibriert werden, indem mehrere Abschnitte der projizierten Linie auf dem weitgehend vertikal orientierten markenlosen Feld der Haltestege oder Pfosten von der Kamera oder den Kameras des Messkopfes erfasst und in bekannter Weise durch eine Funktion, z.B. durch eine Polynomfunktion, beschrieben werden. Diese nicht-lineare Lichtschnittfunktion wird dann bei der 3D Rekonstruktion eingesetzt.

Konkret wird eine Tragplatte, auf der eine waagerechte ebene Stehfläche gebildet ist und die mindestens einen im Wesentlichen senkrecht aufragenden Pfosten trägt, um eine senkrechte Achse gedreht. Wenigstens ein stationärer Triangulationsmesskopf, bestehend aus wenigstens einer Kamera und wenigstens einem Lichtlinienprojektor, projiziert im Wesentlichen zur Ebene der Tragplatte senkrechte Lichtlinien auf eine Person, die auf der Stehfläche der Tragplatte aufrecht steht. Codierte, photogrammetrisch auswertbare Marken sind auf der Tragplatte um die Stehfläche und an dem Pfosten oder einer mit der Tragplatte verbundenen im Wesentlichen senkrechten Struktur angeordnet. Zu einem Rechner werden die während der Drehung der Tragplatte von der Kamera aufgenommenen Bilder übertragen, und der Rechner bestimmt mit einem photogrammetrischen Programm die Raumform der Person oder eines Teils derselben. Dabei wird wenigstens ein markierungsfreies Feld auf der Tragplatte und wenigstens ein markierungsfreies Feld an dem Pfosten oder der daran befestigten Struktur angeordnet. Die Lage der markierungsfreien Felder ist relativ zu den photogrammetrischen Marken so gewählt, dass sie anhand der photogrammetrischen Marken eindeutig bestimmt werden kann. Insbesondere sind die markierungsfreien Felder von den photogrammetrischen Marken umgeben, oder sie grenzen an diese an. Die Lage der markierungsfreien Feldern relativ zu den umgebenden oder angrenzenden photogrammetrischen Marken ist dem Rechner bekannt, z.B. in Form einer Liste oder Tabelle der Marken, welche Begrenzungsmarken für die markierungsfreien Felder sind. Der Rechner bestimmt anhand der Lage der photogrammetrischen Marken sowie Lage und Form der Lichtspuren auf den markierungsfreien Feldern die Parameter des Triangulationsmesskopfes und nimmt so eine Selbstkalibrierung des Erfassungsprozesses vor.

Bei Verwendung von mehreren Triangulationsmeßköpfen wird ein Messraum von der Tragplatte bis mindestens zum höchsten zu erfassenden Körperpunkt der Person jeweils mit Überlappung abgedeckt, wobei jeder Triangulationsmesskopf so ausgerichtet wird, dass er mindestens einen Teil der Marken der Tragplatte und des Pfostens oder der damit verbundenen Struktur erfasst.

Vorteilhaft beleuchten die Linienprojektoren mehrerer Triangulationsmeßköpfe die Person in unterschiedlichen Spektralbereichen, und die jeweils zugehörige Kamera erfasst durch ein optisches Filter nur Licht aus diesem Spektralbereich.

Insbesondere beleuchten die Linienprojektoren mehrerer Triangulationsmeßköpfe die Person in unterschiedlichen Spektralbereichen, und der Rechner ordnet über ein Farbklassifikationsprogramm die Lichtspuren der einzelnen Linienprojektoren im Bild der zugehörigen farbtüchtigen Kamera den Linienprojektoren zu.

Eine weitere Option besteht darin, dass die Linienprojektoren der mehreren Triangulationsmessköpfe über eine Pulsvorrichtung zu unterschiedlichen Zeiten kurzzeitig eingeschaltet werden und eine Synchronisierungseinrichtung diese Zeiten mit dem Bildwechsel der Kameras synchronisiert.

Weitere Einzelheiten der Erfindung ergeben sich aus der folgenden ausführlichen Beschreibung unter Bezugnahme auf die beigefügten Zeichnungen. In den Zeichnungen zeigen:
Fig. 1 eine schematische Seitenansicht einer 3D-Erfassungsanordnung im Gebrauch;
Fig. 2 eine schematische Seitenansicht einer Weiterbildung der 3D-Erfassungsanordnung;
Fig. 3 eine perspektivische Schrägansicht zur Veranschaulichung eines Erfassungsvorgangs;
Fig. 3a eine perspektivische Schrägansicht zur Veranschaulichung der Lage von markierungsfreien Feldern relativ zu umgebenden oder angrenzenden photogrammetrischen Marken;
Fig. 4 eine Skizze zur Erläuterung eines Aspektes der inhärenten Kalibrierung;
Fig. 5 eine schematische Perspektivansicht eines Triangulationsmeßkopfes; und
Fig. 6 eine weitere Skizze zur Erläuterung eines anderen Aspektes der inhärenten Kalibrierung.

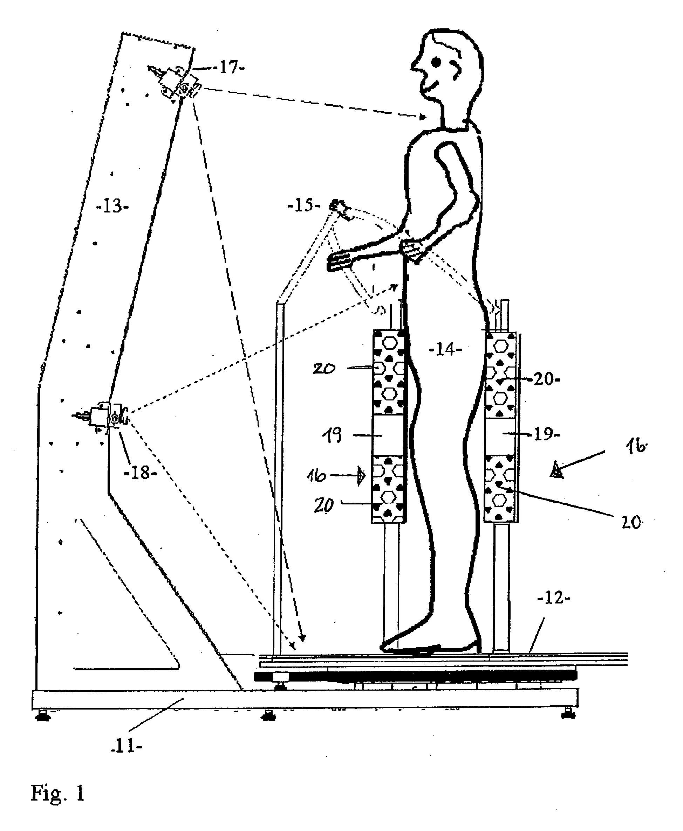

Fig. 1 zeigt in einer vereinfachten Darstellung den mechanischoptischen Aufbau des Erfassungsanordnung 11. Diese umfasst eine kreisförmige ebene und vorzugsweise motorisch angetriebene Tragplatte 12 mit einer Stehfläche, um die herum die Tragplatte in einer speziellen Weise mit photogrammetrischen Marken versehen ist. Ein Kunde 14 steht aufrecht auf dieser Stehfläche der Tragplatte, im Folgenden auch als "Drehteller" bezeichnet. Eine Haltevorrichtung, an der sich der Kunde 14 während der Drehbewegung festhält, besteht aus einem Haltebügel 15 und zwei senkrecht von dem Drehteller aufragenden Pfosten 16. Ferner enthält die Anordnung einen Messarm 13, welcher beispielsweise zwei Kamera/Laserlinienprojektor-Messköpfe 17, 18 so ausrichtet, dass beide sowohl einen Teil des Körpers des Kunden 14 als auch einen Teil der sich drehenden photogrammetrisch markierten Fläche des Drehtellers 12 erfassen. Die Pfosten 16 sind ebenfalls in einer speziellen Weise mit photogrammetrischen Marken versehen. Diese Markierung besteht aus zwei Teilflächen 20 mit photogrammetrisch auswertbaren Marken und einer markenlosen Teilfläche 19. Anstatt der Pfosten 16 können andere im wesentlichen senkrechte und mit der Tragplatte 12 oder den Pfosten 16 starr verbundene Strukturen verwendet werden, um daran die Teilflächen 20 mit photogrammetrisch auswertbaren Marken und die markenlose Teilfläche 19 anzuordnen.

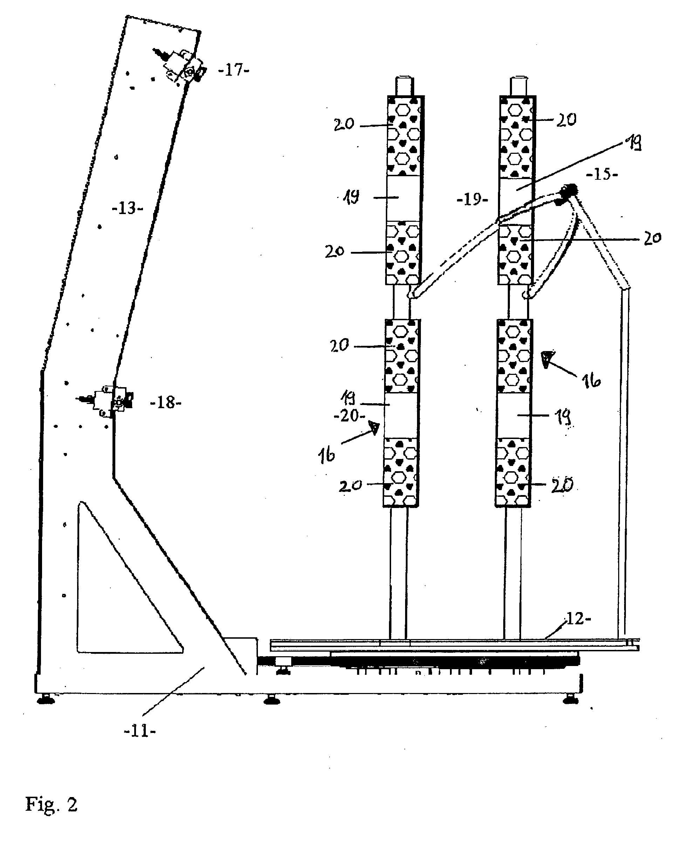

Fig. 2 zeigt eine Variante des Grundaufbaus aus Fig. 1, bei welchem die photogrammetrische Markierung der Pfosten 16 oberhalb der Taille des Kunden 14 wiederholt wird, um zur erfindungsgemäßen inhärenten Selbstkalibrierung insbesondere des oberen Kamera/Laserlinien-Messkopfes 17 vorteilhaft plazierte photogrammetrische Marken zu erfassen.

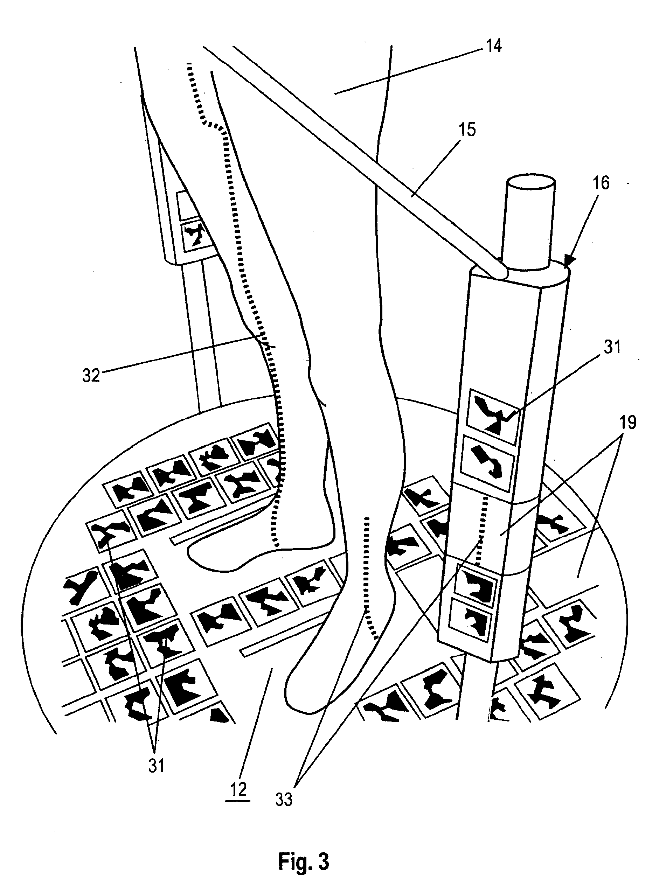

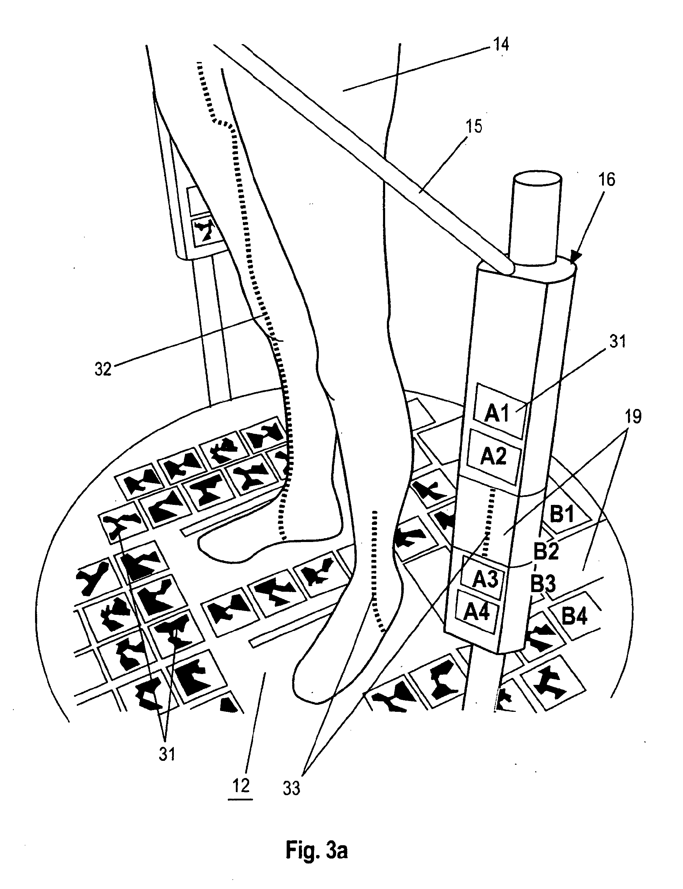

Fig. 3 zeigt eine Aufsicht auf die photogrammetrisch markierte Grundfläche des Drehtellers 12 sowie auf die Markierung des Pfostens 16, wobei erfindungsgemäß zwei grundsätzlich verschiedene Markierungsarten erkennbar sind, nämlich eine größere Anzahl von absolut codierten photogrammetrisch auswertbaren Marken 31 sowie einige freie, markenlose Felder 19 innerhalb der Felder von photogrammetrischen Marken, wobei in diesen freien Feldern 19 der Verlauf der Lichtspuren des oder der Linienprojektoren ungestört von Marken erkennbar ist. Außerdem ist beispielhaft die Lichtspur 32 eines Linienprojektors des Messkopfes auf dem rechten Bein angedeutet sowie ein Teil der Lichtspur 33 eines zweiten Linienprojektors auf dem linken Fuß- und Wadenbereich.

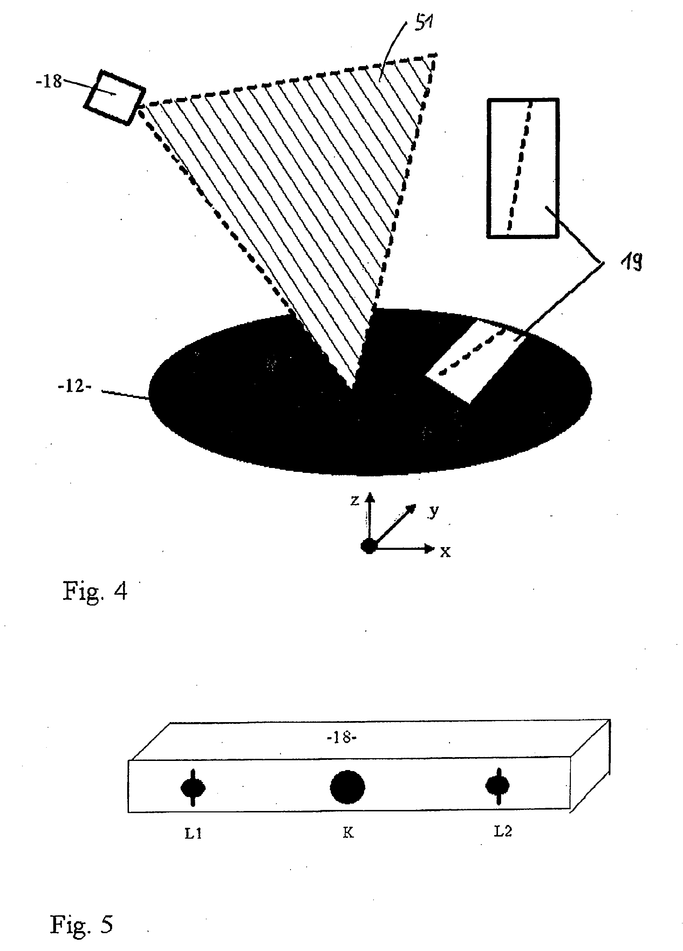

Fig. 4 zeigt in einer auf die wesentlichen Elemente reduzierten Skizze den Triangulationsmesskopf 18, den photogrammetrisch markierten (schwarz dargestellt) motorisch angetriebener Drehteller 12 sowie eine projizierte Lichtebene 51. Diese Skizze verdeutlicht, wie anhand des Verlaufs der Lichtspuren des Linienprojektors in dem von photogrammetrischen Marken umrandeten markenlosen Feld des Drehtellers die Verdrehung des Linienprojektors in der XY-Ebene und in dem vertikalen von photogrammetrischen Marken umrandeten markenlosen hellen Feld des Pfostens die Verkantung des Linienprojektors in der ZX-Ebene sichtbar und damit aus dem Kamerabild auswertbar sind und somit alle Daten bereitstehen, um die inneren und äußeren Parameter der Triangulationsanordnung von Kamera und Linienprojektor photogrammetrisch automatisch zu bestimmen und damit den gesamten Messkopf inhärent re-kalibrierbar zu gestalten.

Fig. 5 zeigt beispielhaft einen Messkopf 18, bestehend aus einer Kamera K und zwei rechts und links hiervon symmetrisch in einem Basisabstand d angebrachten Lichtlinien-Projektoren L1 und L2, welche fortlaufend den Messraum mit diesen Linienstrukturen ausleuchten.

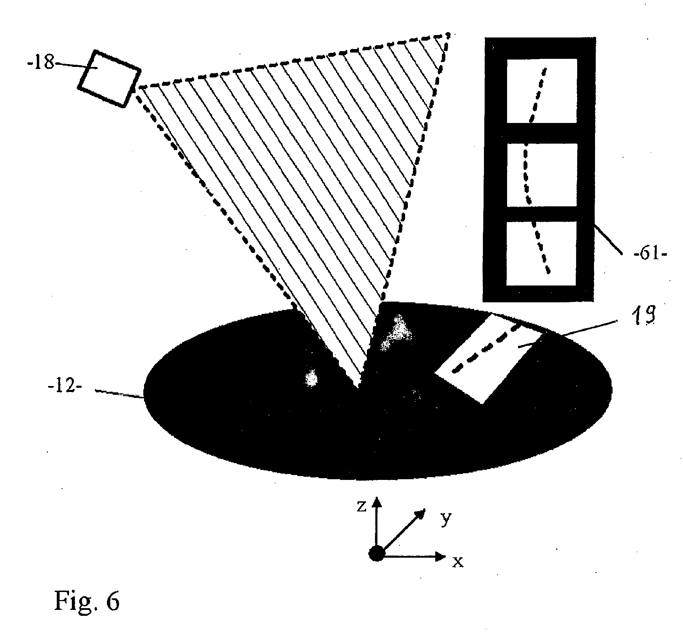

Fig. 6 zeigt beispielhaft, wie auch Abweichungen von der Geradlinigkeit des Linienprojektors dadurch erkannt werden, dass wesentliche Abschnitte der auf die markenlose, vorwiegend vertikal angeordnete und von Markierungen umrandete Fläche 61 projizierten Lichtspur mit der Kamera im Messkopf 18 aufgenommen werden und der nicht-geradlinige Verlauf durch ein glatte Approximationsfunktion wie z.B. eine Polynomfunktion modelliert wird.

Der Erfindungsgedanke sei anhand einer Anordnung beschrieben, welcher einen Ganzkörper-Scanner betrifft mit einem Messraum, welcher von den Füßen bis zur Halsregion des Kunden reicht, wobei mit zwei vertikal gestapelten Triangulationsmessköpfen gearbeitet wird. Diese bestehen beispielhaft und wie in Fig. 4 skizziert jeweils aus einer Kamera K und zwei links und rechts im Abstand d davon angeordneten vertikal abstrahlenden Linienprojektoren L1 und L2. Diese spezielle Auslegung wird aus didaktischen Gründen gewählt und ist nicht einschränkend zu verstehen. Der Erfindungsgedanke umfasst auch Körperscanner mit einem anderen kleineren oder größeren Bildfeld sowie mit nur einem einzigen oder aber mehr als zwei Triangulationsmessköpfen sowie mit Triangulationsmessköpfen mit mehr als einer Kamera und/oder mehr als zwei Linienprojektoren.

Solche Triangulationsmessköpfe sind dem Fachmann der Bildverarbeitung bekannt. Als Linienprojektoren werden in der Regel Halbleiterlaser eingesetzt mit einer vorgesetzten Optik zur Erzeugung einer geradlinigen und möglichst scharf begrenzten Lichtspur.

Wie in Fig. 1 gezeigt, besteht die erfindungsgemäße kalibrationsfreie bzw. inhärent selbstkalibrierende (beide Begriffe werden im Rahmen dieser Beschreibung als synonym verwendet) Erfassungsanordnung 11 aus folgenden Grundelementen:
1) einer starren mechanischen Konstruktion zur Aufnahme eines motorisch angetriebenen Drehtellers 12, auf welchem der zu scannende Kunde 14 aufrecht steht und sich mit Hilfe eines Haltebügels 15 festhält, einer Haltekonstruktion bzw. einem 13 zur Aufnahme und starren Ausrichtung von beispielsweise zwei Triangulationsmessköpfen 17, 18 (Kamera/Laserlinienprojektor), deren Abtasträume so ausgerichtet sind, dass sie sich teilweise überlappen und dass dabei beide zusätzlich zum Körper des Kunden einen Teilausschnitt des photogrammetrisch markierten Drehtellers und der photogrammetrisch markierten Pfosten 16 erfassen, wobei der obere Messkopf 17 im wesentlichen die oberen Teile des Körpers erfasst und die okkludierten Teile wie beispielsweise den Unterbauch nicht erfasst, der untere Messkopf 18 im wesentlichen die unteren Teiles des Körpers und die vom oberen Messkopf 17 nicht sichtbaren okkludierten Teile erfasst.
2) die beiden vertikalen Pfosten 16 mit dem Haltebügel 15, welche erfindungsgemäß in zwei unterschiedlichen Arten markiert sind:
   a. mit absolut codierten photogrammetrisch auswertbaren Marken 31
   b. mit nicht codierten Freiflächen 19.

Fig. 2 zeigt eine weitere Ausgestaltungsform der codierten Pfosten 16, bei welcher sowohl die sich unterhalb des Haltebügels 15 befindenden Teile der Pfosten 16 codiert sind als auch zwei weitere Teile der Pfosten 16 in den oberen Messraum hineinragen und in gleicher Weise markiert und codiert sind. Hierdurch werden zur erfindungsgemäßen inhärenten Selbstkalibrierung insbesondere des oberen Messkopfes 17 vorteilhaft angeordnete markierte Flächen erzeugt.

Fig. 3 zeigt in einer drucktechnisch bedingten s/w Feinrasterdarstellung im Detail die Ausgestaltung der erfindungsgemäßen Markierung sowohl des Drehteller 12 als auch der vertikalen Pfosten 16. Sichtbar sind zwei projizierte Lichtlinien des Messkopfes, wobei die Linie 32 des ersten Linienprojektors L1 des Messkopfes in dieser Aufnahme im wesentlichen das rechte Bein und den Unterbauch hell markiert und die Linie 33 des zweiten Linienprojektors L2 des Meßkopfes den Waden-/Fuß-Bereich des linken Beines markiert und ebenfalls auf dem freien Feld 19 des Pfostens 16 eine gut sichtbare Linienspur hinterlässt.

Die photogrammetrischen Markierungen 31 des Drehtellers definieren die XY-Grundfläche des Weltkoordinatensystem des Scanners. Sie werden bei jeder Aufnahme während des Drehens des Kunden zusammen mit den Lichtlinienspuren von der Kamera erfasst und erlauben damit, wie dem Fachmann der Photogrammetrie bekannt, die automatische Bestimmung der inneren und äußeren Parameter der Kamera. Sie bilden außerdem einen absoluten Maßstab zur Umrechnung von lateralen Auslenkungen der Linienspuren in Entfernungsdaten und hieraus die automatische Erstellung eines kalibrierten 3D Modells des gescannten Körpers. Damit ist eine inhärente Selbstkalibrierung der Kamera und ihrer Halterung erreicht, nicht allerdings die Kalibrierung des kompletten Messkopfes bestehend, wie in Fig.4 beispielhaft gezeigt, aus einer Kamera K und zwei symmetrisch Linienprojektoren L1 und L2 im Basisabstand d und mit einem vorgegebenen Triangulationswinkel in Bezug auf die optische Achse der Kamera ausgerichtet. Es können mit dieser Anordnung noch keine Angaben zur Kalbrierung des gesamten Messkopfes, d.h. zur automatischen Bestimmung der inneren und äußeren Parameter des gesamten Messkopfes gewonnen werden.

Wie Fig.3 zeigt, ist erfindungsgemäß zu diesem Zwecke die Markierung des Drehtellers beispielhaft an mehreren Stellen durch eine markenlose Fläche 19 unterbrochen; auch die Markierung der Pfosten 16 ist durch eine solche markenlose Fläche 19 unterbrochen. Diese markenlosen Flächen 19 auf dem Drehteller 12 und den Pfosten 16 dienen wie anschließend erläutert, in einer erfindungsgemäßen Weise zur inhärenten Selbstkalibrierung des gesamten Messaufbaus, bestehend aus Kameras, Linienprojektoren und mechanischen Aufbauten einschließlich der (markierten) Pfosten.

Dieser grundlegende Erfindungsgedanke sei näher anhand der Skizze Fig. 4 erläutert. Um den Messkopf 18, bestehend aus beispielsweise einem Linienprojektor und einer Kamera zu kalibrieren, müssen alle inneren und äußeren Parameter nicht nur der Kamera, sondern des gesamten Messkopfes photogrammetrisch mit Hilfe der während des Drehvorgangs erstellten Aufnahmen berechnet werden können.

Die Bestimmung der inneren und äußeren Parameter der Kamera kann in bekannter Weise aus der Sequenz der Aufnahmen während des Drehvorgangs geschehen, da der markierte Drehteller zumindest teilweise in jeder Aufnahme sichtbar ist.

Erfindungsgemäß werden die äußeren und die inneren Parameter des oder der Linienprojektoren dadurch bestimmt, dass in zahlreichen Aufnahmen während der Drehbewegung die linienhaften Lichtspuren der Linienprojektoren anhand der unmarkierten Teilflächen 19 auf dem Drehteller und der unmarkierten Teilflächen 19 an den Pfosten 16 ausgewertet werden.

Der Verlauf der Lichtspur auf einem unmarkierten Feld des Drehtellers 12 ermöglicht die Ableitung des Triangulationswinkels zwischen Kamera und projizierter Lichtebene in der XY-Ebene, d.h. in Bezug auf die Grundfläche des Weltkoordinatensystems XYZ.

Der Verlauf der Lichtspur auf einem unmarkierten Feld der Pfosten 16 ermöglicht die Bestimmung der Verkantung des Linienprojektors, d.h. des Drehwinkels in der ZX-Ebene.

Allerdings muss hierfür die Lage der unmarkierten Fläche der Pfosten im Raum bekannt sein. Dies kann beispielsweise durch eine streng vertikale Ausrichtung entlang der Z-Achse geschehen. Eine solche Forderung widerspricht allerdings dem Ziel eines preiswerten Scanners mit einer lediglich moderat robusten und konstanten mechanischen Konstruktion.

Erfindungsgemäß wird daher die Lage dieser nicht-markierten Fläche(n) im Raum mit Hilfe der absoluten Markierungen 31 (siehe Fig. 3) der Pfosten 16 photogrammetrisch aus den Kamerabildern während des Drehvorgangs ermittelt, so dass die gesamte Haltekonstruktion bestehend aus Haltebügel 15 und Pfosten 16 ebenfalls mechanisch nicht genau und konstant konstruiert werden muss, sondern ihre Genauigkeit aus der inhärenten Bestimmung der Raumlage der nicht-markierten Teilflächen der Pfosten photogrammetrisch abgeleitet werden kann. Dabei wird vorausgesetzt, dass die Lage der markierungsfreien Feldern relativ zu den umgebenden oder angrenzenden photogrammetrischen Marken dem Rechner bekannt ist, z.B. in Form einer Liste oder Tabelle der Marken, welche Begrenzungsmarken für die markierungsfreien Felder sind.

Da die photogrammetrischen Marken durch ihre individuelle Codierung eindeutig identifiziert sind, sind auch die eingebetteten markierungsfreien Felder eindeutig identifiziert. Damit können diese markierungsfreien Felder automatisch im Kamerabild detektiert werden und ebenfalls mit dem Fachmann der Bildverarbeitung und der Photogrammetrie bekannten Verfahren der geometrische Verlauf der mindestens einen Lichtspur des mindestens einen Linienprojektors in dem oder den markierungsfreien Feldern bestimmt werden.

In Fig. 3a wird der in Fig. 3 bereits aufgeführte Gedanke graphisch deutlicher erklärt. Es seien beispielhaft zwei markierungsfreie Felder vorhanden, das Feld A auf dem senkrechten Pfosten und das Feld B auf der waagrechten Grundplatte. Das Feld A ist von den photogrammetrischen Marken A1, A2 (oberhalb) und den Marken A3, A4 (unterhalb) eingerahmt. Das Feld B ist von den Marken B1, B2, B3, B4 eingerahmt, wobei in der Darstellung die Marken B3 und B4 teilweise von dem senkrechten Pfosten verdeckt sind. Die Symbole A und B stehen hierbei für die absolut codierten graphischen photogrammetrischen Marken.

Während der Drehbewegung werden alle sichtbaren photogrammetrischen Marken in den jeweils von der Kamera erfassten Bildern aufgenommen. Hierbei sind auch die markierungsfreien Felder enthalten. Ob die eigentliche photogrammetrische Auswertung bereits parallel zu der Drehbewegung im Auswerterechner passiert oder erst nach einem vollständigen Scan (welcher aus typ. 100 Bildern besteht) ist unerheblich, beide Möglichkeiten sind im Stand der Technik bekannt.

In dem Auswerterechner ist die Festlegung "Form+Ort + umgebende photogrametrische Marken des markierungsfreien Feldes" für jedes markierungsfreie Feld hinterlegt. Die Definition, wo die markierungsfreien Felder liegen, dh. durch welche photogrammetrischen Marken diese begrenzt sind, muß dem Auswerterechner bekannt sein (z.B. in Form einer Liste oder Tabelle der Marken, welche Begrenzungsmarken für markierungsfreie Felder darstellen. Die photogrammetrische Auswertung aller Marken liefert 3D Koordinaten, d.h. die 3D Position jeder Marke und damit auch die 3D Position jedes markierungsfreien Feldes. Nach der photogrammetrischen Auswertung liegt daher fest: (a) WO im Weltkoordinatensystem sich markierungsfreie Felder befinden (Lage im Raum); (b) um WELCHE markierungsfreien Felder es sich handelt (Feld A, Feld B,....), wie sich anhand der Indizes der umgebenden Marken feststellen läßt; (c) und damit implizit auch welche FORM diese Felder haben (rechteckig, quadratisch,...). Durch diese Auswertung kann dann gezielt in den markierungsfreien Bereichen oder Feldern der geometrische Verlauf (Winkel bezogen auf die XY- bzw. auf die ZX Koordinatenachsen) der Lichtspur des mindestens einen Linienprojektors bestimmt werden (sofern die Lichtspur in der betreffenden Aufnahme ein markierungsfreies Feld trifft) . Mit diesen beiden Winkeln ist die Verdrehung und Verkantung des mindestens einen Lichtlinienprojektors bezüglich des Weltkoordinatensystems bestimmt und damit das gesamte Triangulationssystem Kamera+Linienprojektor inhärent kalibrierbar, d.h. im Sinne dieser Erfindung für den Anwender kalibrationsfrei.

Auch Abweichungen in der Geradlinigkeit der projizierten Linie, beispielsweise durch Fehler oder Dejustagen der Strahlformungsoptiken des Linienprojektors, können nach Fig. 6 kalibriert werden, indem mehrere Abschnitte der auf das weitgehend vertikal orientierte markenlose Feld 61 projizierten Linie von der Kamera oder den Kameras des Messkopfes erfasst werden und in bekannter Weise durch eine Funktion, z.B. durch eine Polynomfunktion, beschrieben werden. Diese nicht-lineare Lichtschnittfunktion wird dann bei der 3D Rekonstruktion eingesetzt.

Damit sind erfindungsgemäß der gesamte mechanisch Messaufbau und die Messköpfe bestehend aus Kamera(s) und Linienprojektor(en) inhärent selbstkalibrierend, d.h. aus Sicht des Anwenders kalibrationsfrei:
c. die inneren und äußeren Parameter der mindestens einen Kamera eines Messkopfes werden aus der Beobachtung der photogrammetrischen Markierungen des Drehtellers während der Drehbewegung abgeleitet;
d. die noch fehlenden inneren und äußeren Parameter der Linienprojektoren im mindestens einen Triangulationsmesskopf werden aus der Beobachtung des Verlaufs der Lichtspuren des mindestens einen Lichtlinienprojektors in den markierungsfreien Feldern des Drehtellers und der Pfosten abgeleitet.

Der Aufbau des Scanners kann damit entsprechend leicht und kostengünstig sein, so dass das erfindungsgemäße Ziel eines Ganzkörperscanners, welcher sowohl kostengünstig herzustellen ist als auch einfach ohne wiederholte komplizierte Kalibrierungsverfahren zu bedienen ist, erreicht wird.

Der Aufbau nach Fig.1 sowie die Konstruktion des Triangulationsmesskopfes nach Fig. 4 sind lediglich beispielhaft zu verstehen. Auch Aufbauten mit nur einem Triangulationsmesskopf oder mit mehr als zwei Triangulationsmessköpfen, mit einer anderen als der gezeigten Anordnung des oder der Messköpfe sind vom Erfindungsgedanken erfasst, solange die Messköpfe während des Scanvorgangs sowohl die photogrammetrisch markierten als auch die markierungsfreien Felder des Drehtellers und der Pfosten erfassen.

Die Anbringung der vertikal angebrachten Markierungsfelder an den Pfosten 16 ist beispielhaft zu verstehen. Anordnungen, bei welchen diese Felder getrennt von einer Haltevorrichtung angebracht sind und sich mit dem Drehteller bewegen, werden vom Erfindungsgedanken erfasst.

Erfindungsgemäß werden bei Messköpfen mit mehr als einem Linienprojektor bzw. bei der Verwendung von mehreren Messköpfen mit jeweils einem oder mehreren Linienprojektoren diese Projektoren zur besseren Zuordnung ihrer Lichtspuren zu den Kamerabildern entweder im Spektral- oder Zeit-Multiplex betrieben.

Unter "Spektral-Multiplex" wird die Vorgabe verstanden, dass jeder Projektor eine Linie mit einer unterschiedlichen Grundfarbe im Spektralbereich der Kamera-Empfindlichkeit ausstrahlt. Durch Verwendung einer farbtüchtigen Kamera können damit die Spuren unterschiedlicher Linienprojektoren anhand der jeweils unterschiedlichen Farbe unterschieden werden.

Unter "Spektral-Multiplex" wird im Rahmen dieser Erfindung auch die Tatsache verstanden, dass die unterschiedlichen Linienprojektoren in einem jeweils unterschiedlichen Wellenlängen-Bereich ausstrahlen und die Kameras der einzelnen Messköpfe jeweils mit Hilfe eines optischen Bandpassfilters nur für die Lichtlinie eines Projektors empfindlich sind. Damit lassen sich beispielsweise in einfacher Weise die Lichtspuren des oberen Messkopfes 17 aus Fig. 1 von denen des unteren Messkopfes 18 im Überlappungsbereich der Kameras beider Messköpfe trennen.

Unter "Zeit-Multiplex" wird im Rahmen dieser Erfindung die Tatsache verstanden, dass die unterschiedlichen Linienprojektoren nur kurzzeitig zu unterschiedlichen Kamera-Bildaufnahmen gepulst betrieben werden, so dass die von den einzelnen Linienprojektoren erzeugten Lichtspuren sich in einem Kamerabild nicht überlagern.

Es ist dem Fachmann der Bildverarbeitung bekannt, Spektral- und Zeit-Multiplex Anordnungen zu mischen, um den besten Kompromiss zwischen der gleichzeitigen Ausstrahlung möglichst vieler Lichtlinien und der fehlerfreien Zuordnung dieser Lichtlinien zu den einzelnen Projektoren aus den Kamerabildern zu ermöglichen.

Durch die Kalibrationsfreiheit ist der erfindungsgemäße Scanner auch leicht modular zu erweitern. So kann die kostengünstige Eintrittsvariante z.B. nur einen einzigen Messkopf enthalten, welcher einen eingeschränkten Messraum von den Füssen bis zur Taille reichend abdeckt. Die spätere Erweiterung durch Anbringung eines weiteren Messkopfes ist leicht durchzuführen, da durch die Selbstkalibrierung keine genauen konstruktiv sicherzustellenden Ausrichtungen, Objektiveinstellungen etc. erforderlich sind. Damit sind die Voraussetzungen für ein hochmodulares leicht erweiterbares 3D Messsystem gegeben.

Erfindungsgemäß wird der 3D Körperscanner durch zusätzliche Sensoren und Signalgeber zu einem sog. multisensoriellen System ausgebaut, welches neben der 3D Raumform zusätzliche medizinisch-diagnostisch interessante Informationen erzeugt und oder Reize zur Therapie in den Körper einkoppelt. Hierfür ist der gesamte Aufbau bereits zur Aufnahme zusätzlicher Sensoren vorbereitet und die Software zur Einbindung der entsprechenden Zusatzprogramme ausgestaltet.

Dieser Erfindungsgedanke sei beispielhaft anhand der folgenden, nicht ausschließlichen Liste von Zusatzmodulen und -messungen verdeutlicht:
1. über eine in vorbereitete Kammern im Drehteller nachrüstbare Waage bzw. Wägezellen kann zusätzlich zur Raumform das Körpergewicht bestimmt werden;
2. über eine für jeden Fuß getrennte Waage bzw. Drucksensor wird die Gleichgewichtslage des stehenden Kunden bestimmt;
3. über eingebaute ortsaufgelöste Drucksensoren werden die Sohlenabdruck-Bilder der Füße des Kunden ermittelt und damit zusätzlich wichtige Daten für die Herstellung einer anatomisch passenden Einlage bereitgestellt;
4. über elektrodynamische Feldelektroden an den Fußsohlen wird der Fettanteil des Körpers näherungsweise bestimmt;
5. über Hand-Elektroden am Haltebügel wird der Puls gemessen;
6. über Hand- und Fußelektroden wird das Körperfettanteil elektrodynamisch gemessen;
7. in der Fußposition im Drehteller und/oder in der Handposition im Haltebügel werden aktiv schaltbare Leuchtquellen zu diagnostischen Zwecken eingebaut, welche spektral abgestimmtes Licht über die Fußsohle oder über die Hände in den Körper des Kunden einkoppeln; die erzeugten Leuchteffekte in Händen und Beinen werden mit der oder den Kameras des oder der Messköpfe beobachtet, um medizinisch-diagnostische Aussagen über Durchblutung, Gewebedichte, Arterien- und Venenzustand u.ä zu erfassen;
8. über in vorbereitete Halterungen zusätzlich nachrüstbare Farbkameras wird ein für dermatologische Zwecke nützliches Farbbild zusätzlich zum 3D Modell erzeugt und getrennt oder dem Modell überlagert dargestellt;
9. über die vorhandenen Kameras der Messköpfe wird neben dem Verlauf der Lichtlinien auf der Körperoberfläche auch die laterale Lichtstreuung (der erzeugte Lichthof um die Linie herum) gemessen und als Maß für lokale medizinisch relevante Besonderheiten wie Vernarbungen, Hautflechten und -tumore, hautnahe Flüssigkeitsansammlungen und ähnlich sich in der lokalen Lichtstreuung ausdrückende medizinisch relevante Besonderheiten erfasst;
10. über eine zusätzliche Wärmebildkamera werden diagnostisch relevante Wärmebilder gewonnen und getrennt oder dem 3D Modell überlagert dargestellt;
11. über einen oder mehrere im Drehteller und/oder in dem Haltebügel eingebaute Bewegungsdetektoren wird erkannt, ob sich der Kunde während des Drehens des Drehtellers in einer unzulässigen Weise bewegt hat und damit einen unbrauchbaren Datensatz erzeugt, so dass der Scanvorgang abgebrochen werden kann;
12. über ein Steuerprogramm wird bei einem Abbruch wie vorstehend unter 11. genannt eine Markierung im 3D Datensatz erzeugt, und bei der späteren Rekonstruktion des gesamten 3D Modells werden die einzelnen Ansichten nur in den Anschnitten rekonstruktuiert, welche einer bewegungslosen Abtastung entsprechen, und in einem zweiten Schritt werden diese getrennten Teilansichten zu einem vollständigen 3D Modell eingepasst.

Erfindungsgemäß werden nicht nur Sensoren nachgerüstet, sondern aktiv physikalische Signale oder Reize in den Körper des zu scannenden Kunden eingespeist, um zusätzlich zur Raumform medizinisch/diagnostisch interessante Daten abzuleiten. Auch hier sind diese Aktuatoren erfindungsgemäß modular nachrüstbar, Beispielhaft werden folgende aktive Signaleinspeisungen genannt:
13. niederfrequent gepulste Magnetfelder zur Arthrose- und Knochenheilungs- Behandlung werden über im Drehteller und oder in dem Haltebügel eingebaute Spulen eingekoppelt;
14. mittelfrequente mechanische Vibrationssignale zum Muskeltraining, zur Tinnitus-Behandlung usw. werden über im Drehteller und oder in dem Haltebügel eingebaute Spulen eingekoppelt.

Der Erfindungsgedanke umfasst somit neben einem besonders kostengünstigen und leicht zu bedienenden kalibrationsfreien 3D Körper- oder Körperteil-Scanner dessen modularen Ausbau zu einer multisensoriellen Mess-und Diagnose- und Therapiestation mit der Möglichkeit der getrennten oder der Raumform überlagerten medizinisch/diagnostisch nützlichen Daten.

## Patentansprüche

1. Verfahren zur kalibrationsfreien kostengünstigen und genauen optischen Erfassung der Raumform von Körpern und Körperteilen nach dem Lichtschnitt-Verfahren, mit den folgenden Schritten:
a) eine Tragplatte (12), auf der eine waagerechte ebene Stehfläche gebildet ist und die mindestens einen im Wesentlichen senkrecht aufragenden Pfosten (16) oder eine im wesentlichen senkrechte mit der Tragplatte verbundene Struktur trägt, wird um eine senkrechte Achse gedreht, während eine Person (14) auf der Stehfläche der Tragplatte aufrecht steht;
b) wenigstens ein stationärer Triangulationsmesskopf (17, 18), bestehend aus wenigstens einer Kamera (K) und wenigstens einem Lichtlinienprojektor (L1, L2), projiziert im Wesentlichen zur Ebene der Tragplatte (12) senkrechte Lichtlinien auf die Person (14);
c) codierte, photogrammetrisch auswertbare Marken (31) sind auf der Tragplatte (12) um die Stehfläche und an dem Pfosten (16) oder der im Wesentlichen senkrechten Struktur angeordnet;
d) zu einem Rechner werden die während der Drehung der Tragplatte (12) von der Kamera (K) aufgenommenen Bilder übertragen, und der Rechner bestimmt mit einem photogrammetrischen Programm die Raumform der Person oder eines Teils derselben;
wobei
e1) wenigstens ein markierungsfreies Feld (19) auf der Tragplatte (12) und wenigstens ein markierungsfreies Feld (19) an dem Pfosten (16) oder der im wesentlichen senkrechten Struktur angeordnet und im Rechner die Lage des wenigstens einen markierungsfreien Feldes relativ zu den umgebenden oder angrenzenden photogrammetrisch auswertbaren Marken (31) hinterlegt ist,
e2) und der Rechner anhand der Lage der photogrammetrisch auswertbaren Marken (31) sowie Lage und Form der Lichtspuren auf den markierungsfreien Feldern (19) die Parameter des Triangulationsmesskopfes (17, 18) bestimmt und eine Selbstkalibrierung des Erfassungsgeräts vornimmt, wobei für die markierungsfreien Felder (19) anhand der Lage der photogrammetrisch auswertbaren Marken (31) und der im Rechner hinterlegten Lage der markierungsfreien Felder relativ zu den umgebenden oder angrenzenden photogrammetrisch auswertbaren Marken (31) eine Lage im Raum bestimmt wird.

2. Verfahren nach Anspruch 1, **dadurch gekennzeichnet, dass** mehrere Triangulationsmeßköpfe (17, 18) einen Messraum von der Tragplatte (12) bis mindestens zum höchsten zu erfassenden Körperpunkt jeweils mit Überlappung abdecken, und jeder Triangulationsmesskopf (17, 18) so ausgerichtet wird, dass er mindestens einen Teil der Marken (31) der Tragplatte (12) und des Pfostens (16) oder der im wesentlichen senkrechten Struktur erfasst.

3. Verfahren nach einem der Ansprüche 1 und 2, **dadurch gekennzeichnet, dass** die Linienprojektoren mehrerer Triangulationsmeßköpfe in unterschiedlichen Spektralbereichen emittieren und der jeweils zugehörigen Kamera ein optisches Filter vorgeschaltet wird, das nur für Licht aus diesem Spektralbereich durchlässig ist.

4. Verfahren nach einem der Ansprüche 1 und 2, **dadurch gekennzeichnet, dass** die Linienprojektoren mehrerer Triangulationsmeßköpfe in unterschiedlichen Spektralbereichen emittieren und die jeweils zugehörige Kamera farbtüchtig ist und der Rechner ein Farbklassifikationsprogramm ausführt, das die Lichtspuren der einzelnen Linienprojektoren im Bild der Farbkamera den Linienprojektoren zuordnet.

5. Verfahren nach einem der Ansprüche 1 bis 4, **dadurch gekennzeichnet, dass** mittels in der Tragplatte und/oder der Haltevorrichtung eingebauten Sensoren durch die Person erzeugte Signale erfasst werden, die der Rechner auswertet.

6. Verfahren nach einem der Ansprüche 1 bis 5, **dadurch gekennzeichnet, dass** mittels in der Tragplatte und/oder der Haltevorrichtung eingebauten Aktuatoren durch die Person wahrnehmbare physikalische Reize erzeugt werden.

7. Erfassungsgerät zur kalibrationsfreien kostengünstigen und genauen optischen Erfassung der Raumform von Körpern und Körperteilen nach dem Lichtschnitt-Verfahren, insbesondere zur Durchführung des Verfahrens nach einem der Ansprüche 1 bis 6, **gekennzeichnet durch**
a) eine um eine senkrechte Achse drehbare Tragplatte (12), auf der eine waagerechte ebene Stehfläche gebildet ist und die mindestens einen im Wesentlichen senkrecht aufragenden Pfosten (16) oder eine im wesentlichen senkrechte mit der Tragplatte verbundene Struktur trägt;
b) wenigstens einen stationären Triangulationsmesskopf (17, 18), bestehend aus wenigstens einer Kamera (K) und wenigstens einem Lichtlinienprojektor (L1, L2), der bei Gebrauch im Wesentlichen zur Ebene der Tragplatte (12) senkrechte Lichtlinien auf eine Person (14) projiziert, die auf der Stehfläche der Tragplatte (12) aufrecht steht;
c) codierte, photogrammetrisch auswertbare Marken (31), die auf der Tragplatte (12) um die Stehfläche und an dem Pfosten (16) oder der im Wesentlichen senkrechten Struktur angeordnet sind;
d) einen Rechner, zu dem die während der Drehung der Tragplatte (12) von der Kamera (K) aufgenommenen Bilder übertragen werden und der mit einem photogrammetrischen Programm die Raumform der Person oder eines Teils derselben bestimmt;
wobei
e1) wenigstens ein markierungsfreies Feld (19) auf der Tragplatte (12) und wenigstens ein markierungsfreies Feld (19) an dem Pfosten (16) oder der im wesentlichen senkrechten Struktur angeordnet ist, wobei im Rechner die Lage des wenigstens einen markierungsfreien Feldes relativ zu den umgebenden oder angrenzenden photogrammetrisch auswertbaren Marken (31) hinterlegt ist,
e2) und der Rechner anhand der Lage der photogrammetrisch auswertbaren Marken (31) sowie Lage und Form der Lichtspuren auf den markierungsfreien Feldern (19) die Parameter des Triangulationsmesskopfes (17, 18) bestimmt und eine Selbstkalibrierung des Erfassungsgeräts vornimmt, wobei für die markierungsfreien Felder (19) anhand der Lage der photogrammetrisch auswertbaren Marken (31) und der im Rechner hinterlegten Lage der markierungsfreien Felder relativ zu den umgebenden oder angrenzenden photogrammetrisch auswertbaren Marken (31) eine Lage im Raum bestimmt wird.

8. Erfassungsgerät nach Anspruch 7, **dadurch gekennzeichnet, dass** der Pfosten (16) Teil einer Haltevorrichtung ist, an der sich die Person (14) bei der Drehung der Tragplatte (12) festhalten kann.

9. Erfassungsgerät nach Anspruch 7 oder 8, **dadurch gekennzeichnet, dass** die Tragplatte (12) motorisch drehbar ist.

10. Erfassungsgerät nach Anspruch 7 oder 8, **dadurch gekennzeichnet, dass** die Tragplatte (12) manuell drehbar ist.

11. Erfassungsgerät nach einem der Ansprüche 7 bis 9, **dadurch gekennzeichnet, dass** mehrere Triangulationsmeßköpfe (17, 18) vorgesehen sind, die einen Messraum von der Tragplatte (12) bis mindestens zum höchsten zu erfassenden Körperpunkt jeweils mit Überlappung abdecken, wobei die Linienprojektoren der mehreren Triangulationsmeßköpfe in unterschiedlichen Spektralbereichen emittieren und entweder der jeweils zugehörigen Kamera ein optisches Filter vorgeschaltet ist, das nur für Licht aus diesem Spektralbereich durchlässig ist, oder die jeweils zugehörige Kamera farbtüchtig ist und der Rechner ein Farbklassifikationsprogramm ausführt, das die Lichtspuren der einzelnen Linienprojektoren im Bild der Farbkamera den Linienprojektoren zuordnet.

12. Erfassungsgerät nach einem der Ansprüche 7 bis 10, **dadurch gekennzeichnet, dass** in der Tragplatte und/oder der Haltevorrichtung Sensoren zur elektronischen Erfassung von durch die Person erzeugten Signalen, die durch den Rechner auswertbar sind, und/oder Aktuatoren eingebaut sind, die durch die Person wahrnehmbare physikalische Reize erzeugen können.

## Claims

1. A method for calibration-free, cost-efficient and precise optical detection of the three-dimensional shape of a body or body part according to the light section procedure, comprising the following steps:
(a) a support plate (12) on which a horizontal flat standing surface is formed and which carries at least one substantially vertically projecting post (16) or a substantially vertical structure connected with the support plate is rotated about a vertical axis while a person (14) stands upright on the standing surface of the support plate;
(b) at least one stationary triangulation measuring head (17, 18), consisting of at least one camera (K) and at least one light line projector (L1, L2), projects light lines that are substantially perpendicular to the plane of the support plate (12) onto the person (14);
(c) encoded, photogrammetrically evaluatable marks (31) are arranged on the support plate (12) around the standing surface and on the post (16) or on the substantially vertical structure;
(d) the images taken by the camera (K) during rotation of the support plate (12) are transferred to a computer, and the computer determines the three-dimensional shape of the person or of part of the person by means of a photogrammetric program;
wherein
(e1) at least one marking-free field (19) is arranged on the support plate (12) and at least one marking-free field (19) is arranged on the post (16) or on the substantially vertical structure, and the position of the at least one marking-free field relative to the surrounding or adjacent photogrammetrically evaluatable marks (31) is stored in the computer; and
(e2) on the basis of the positions of the photogrammetrically evaluatable marks (31) and the positions and shapes of the light traces on the marking-free fields (19), the computer determines the parameters of the triangulation measuring head (17, 18) and performs a self-calibration of the detection device, a position in space being determined for the marking-free fields (19) on the basis of the positions of the photogrammetrically evaluatable marks (31) and the positions, stored in the computer, of the marking-free fields relative to the surrounding or adjacent photogrammetrically evaluatable marks (31).

2. The method according to claim 1, **characterized in that** a plurality of triangulation measuring heads (17, 18) cover a measuring space from the support plate (12) up to at least the highest body point to be detected, each with an overlap, and each triangulation measuring head (17, 18) is oriented such that it detects at least part of the marks (31) of the support plate (12) and of the post (16) or of the substantially vertical structure.

3. The method according to either of claims 1 and 2, **characterized in that** the line projectors of a plurality of triangulation measuring heads emit in different spectral regions and an optical filter is connected upstream of the respectively associated camera, the optical filter transmitting light only from this spectral region.

4. The method according to either of claims 1 and 2, **characterized in that** the line projectors of a plurality of triangulation measuring heads emit in different spectral regions and the respectively associated camera is color-capable and the computer executes a color classification program which allocates the light traces of the individual line projectors in the image of the color camera to the line projectors.

5. The method according to any of claims 1 to 4, **characterized in that** signals generated by the person are detected by means of sensors built into the support plate and/or the holding device and are evaluated by the computer.

6. The method according to any of claims 1 to 5, **characterized in that** physical stimuli perceptible by the person are generated by means of actuators built into the support plate and/or the holding device.

7. A detection device for calibration-free, cost-efficient and precise optical detection of the three-dimensional shape of a body or body part according to the light section procedure, in particular for carrying out the method according to any of claims 1 to 6, **characterized by**
(a) a support plate (12) that is rotatable about a vertical axis and on which a horizontal flat standing surface is formed and which carries at least one substantially vertically projecting post (16) or a substantially vertical structure connected with the support plate;
(b) at least one stationary triangulation measuring head (17, 18) consisting of at least one camera (K) and at least one light line projector (L1, L2) which, in use, projects light lines that are substantially perpendicular to the plane of the support plate (12) onto a person (14) who stands upright on the standing surface of the support plate (12);
(c) encoded, photogrammetrically evaluatable marks (31) arranged on the support plate (12) around the standing surface and on the post (16) or on the substantially vertical structure;
(d) a computer to which the images taken by the camera (K) during rotation of the support plate (12) are transferred and which determines the three-dimensional shape of the person or of part of the person by means of a photogrammetric program;
wherein
(e1) at least one marking-free field (19) is arranged on the support plate (12) and at least one marking-free field (19) is arranged on the post (16) or on the substantially vertical structure, the position of the at least one marking-free field relative to the surrounding or adjacent photogrammetrically evaluatable marks (31) being stored in the computer; and
(e2) on the basis of the positions of the photogrammetrically evaluatable marks (31) and the positions and shapes of the light traces on the marking-free fields (19), the computer determines the parameters of the triangulation measuring head (17, 18) and performs a self-calibration of the detection device, a position in space being determined for the marking-free fields (19) on the basis of the positions of the photogrammetrically evaluatable marks (31) and the positions, stored in the computer, of the marking-free fields relative to the surrounding or adjacent photogrammetrically evaluatable marks (31).

8. The detection device according to claim 7, **characterized in that** the post (16) is part of a holding device for the person (14) to hold on to during the rotation of the support plate (12).

9. The detection device according to claim 7 or 8, **characterized in that** the support plate (12) is adapted to be rotated by a motor.

10. The detection device according to claim 7 or 8, **characterized in that** the support plate (12) is adapted to be rotated manually.

11. The detection device according to any of claims 7 to 9, **characterized in that** a plurality of triangulation measuring heads (17, 18) are provided that cover a measuring space from the support plate (12) up to at least the highest body point to be detected, each with an overlap, the line projectors of the plurality of triangulation measuring heads emitting in different spectral regions, and either an optical filter being connected upstream of the respectively associated camera, the optical filter transmitting light only from this spectral region, or the respectively associated camera being color-capable and the computer executing a color classification program which allocates the light traces of the individual line projectors in the image of the color camera to the line projectors.

12. The detection device according to any of claims 7 to 10, **characterized in that** sensors for electronically detecting signals that are generated by the person and can be evaluated by the computer and/or actuators adapted to generate physical stimuli perceptible by the person are built into the support plate and/or the holding device.

## Revendications

1. Procédé de détection optique précise de la forme spatiale de corps et de parties corporelles, sans étalonnage et à des coûts faibles, selon la méthode de la coupe optique, comprenant les étapes suivantes :
a) on fait tourner autour d'un axe vertical une plaque de support (12) sur laquelle est formée une surface d'accès plane horizontale et qui porte au moins un poteau (16) redressé sensiblement vertical ou bien une structure sensiblement verticale reliée à la plaque de support, un individu (14) se trouvant debout sur la surface d'accès de la plaque de support ;
b) au moins une tête stationnaire de mesure de triangulation (17, 18) constituée par au moins une caméra (K) et au moins un projecteur de lignes lumineuses (L1, L2), projette sur l'individu (14) des lignes lumineuses sensiblement perpendiculaires au plan de la plaque de support (12) ;
c) des marquages (31) codés et aptes à être évalués par voie photogrammétrique sont disposés sur la plaque de support (12) autour de la surface d'accès et sur le poteau (16) ou sur la structure sensiblement verticale ;
d) les images enregistrées par la caméra (K) pendant la rotation de la plaque de support (12) sont transférées à un ordinateur, et l'ordinateur détermine par un programme photogrammétrique la forme spatiale de l'individu ou d'une partie de celui-ci ;
dans lequel
e1) au moins un champ (19) dépourvu de marquage est prévu sur la plaque de support (12) et au moins un champ (19) dépourvu de marquage est prévu sur le poteau (16) ou sur la structure sensiblement verticale, et la position dudit au moins un champ dépourvu de marquage par rapport aux marquages entourant ou adjacents, aptes à être évalués par voie photogrammétrique, est stockée dans l'ordinateur,
e2) et l'ordinateur détermine les paramètres de la tête de mesure de triangulation (17, 18) en se basant sur la position des marquages (31) aptes à être évalués par voie photogrammétrique ainsi que sur la position et la forme des traces lumineuses sur les champs (19) dépourvus de marquage, et il procède à un auto-étalonnage de l'appareil de détection, une position dans l'espace étant déterminée pour les champs (19) dépourvus de marquage en se basant sur la position des marquages (31) aptes à être évalués par voie photogrammétrique et sur la position stockée dans l'ordinateur des champs dépourvus de marquage par rapport aux marquages (31) entourant ou adjacents aptes à être évalués par voie photogrammétrique.

2. Procédé selon la revendication 1, **caractérisé en ce que** plusieurs têtes de mesure de triangulation (17, 18) recouvrent un espace de mesure allant de la plaque de support (12) au moins jusqu'au point du corps le plus haut à détecter, avec chevauchement respectif, et chaque tête de mesure de triangulation (17, 18) est orientée de telle sorte qu'elle détecte au moins une partie des marquages (31) de la plaque de support (12) et du poteau (16) ou de la structure sensiblement verticale.

3. Procédé selon l'une des revendications 1 et 2, **caractérisé en ce que** les projecteurs de lignes de plusieurs têtes de mesure de triangulation émettent dans différentes plages spectrales, et un filtre optique est disposé en amont de la caméra associée respective, filtre qui n'est transparent que vis-à-vis de la lumière de cette plage spectrale.

4. Procédé selon l'une des revendications 1 et 2, **caractérisé en ce que** les projecteurs de lignes de plusieurs têtes de mesure de triangulation émettent dans différentes plages spectrales, et la caméra associée respective est à couleurs et l'ordinateur exécute un programme de classification de couleurs qui associe aux projecteurs de lignes les traces lumineuses des projecteurs de lignes individuels dans l'image de la caméra couleur.

5. Procédé selon l'une des revendications 1 à 4, **caractérisé en ce que** des signaux engendrés par l'individu sont détectés au moyen de capteurs intégrés dans la plaque de support et/ou dans le dispositif de maintien, et ils sont évalués par l'ordinateur.

6. Procédé selon l'une des revendications 1 à 5, **caractérisé en ce que** des excitations physiques perceptibles par l'individu sont engendrées au moyen d'actionneurs intégrés dans la plaque de support et/ou dans le dispositif de maintien.

7. Appareil de détection optique précise de la forme spatiale de corps et de parties corporelles, sans étalonnage et à des coûts faibles, selon la méthode de la coupe optique, en particulier pour mettre en oeuvre le procédé selon l'une des revendications 1 à 6, **caractérisé par** :
a) une plaque de support (12) rotative autour d'un axe vertical, sur laquelle est formée une surface d'accès plane horizontale et qui porte au moins un poteau (16) redressé sensiblement vertical ou bien une structure sensiblement verticale reliée à la plaque de support,
b) au moins une tête stationnaire de mesure de triangulation (17, 18) constituée par au moins une caméra (K) et au moins un projecteur de lignes lumineuses (L1, L2), qui, pendant l'utilisation, projette sur un individu (14), situé debout sur la surface d'accès de la plaque de support, des lignes lumineuses sensiblement perpendiculaires au plan de la plaque de support (12) ;
c) des marquages (31) codés et aptes à être évalués par voie photogrammétrique qui sont disposés sur la plaque de support (12) autour de la surface d'accès et sur le poteau (16) ou sur la structure sensiblement verticale ;
d) un ordinateur auquel sont transférées les images enregistrées par la caméra (K) pendant la rotation de la plaque de support (12) et qui détermine par un programme photogrammétrique la forme spatiale de l'individu ou d'une partie de celui-ci ;
dans lequel
e1) au moins un champ (19) dépourvu de marquage est prévu sur la plaque de support (12) et au moins un champ (19) dépourvu de marquage est prévu sur le poteau (16) ou sur la structure sensiblement verticale, et la position dudit au moins un champ dépourvu de marquage par rapport aux marquages entourant ou adjacents, aptes à être évalués par voie photogrammétrique, est stockée dans l'ordinateur,
e2) et l'ordinateur détermine les paramètres de la tête de mesure de triangulation (17, 18) en se basant sur la position des marquages (31) aptes à être évalués par voie photogrammétrique ainsi que sur la position et la forme des traces lumineuses sur les champs (19) dépourvus de marquage, et il procède à un auto-étalonnage de l'appareil de détection, une position dans l'espace étant déterminée pour les champs (19) dépourvus de marquage en se basant sur la position des marquages (31) aptes à être évalués par voie photogrammétrique et sur la position stockée dans l'ordinateur des champs dépourvus de marquage par rapport aux marquages (31) entourant ou adjacents aptes à être évalués par voie photogrammétrique.

8. Appareil de détection selon la revendication 7, **caractérisé en ce que** le poteau (16) fait partie d'un dispositif de maintien auquel l'individu (14) peut se tenir pendant la rotation de la plaque de support (12).

9. Appareil de détection selon la revendication 7 ou 8, **caractérisé en ce que** la plaque de support (12) est rotative par voie motrice.

10. Appareil de détection selon la revendication 7 ou 8, **caractérisé en ce que** la plaque de support (12) est rotative par voie manuelle.

11. Appareil de détection selon l'une des revendications 7 à 9, **caractérisé en ce qu'**il est prévu plusieurs têtes de mesure de triangulation (17, 18) qui recouvrent un espace de mesure allant de la plaque de support (12) au moins jusqu'au point du corps le plus haut à détecter, avec chevauchement respectif, dans lequel les projecteurs de lignes de plusieurs têtes de mesure de triangulation émettent dans différentes plages spectrales, et soit un filtre optique est disposé en amont de la caméra associée respective, filtre qui n'est transparent que vis-à-vis de la lumière de cette plage spectrale, soit la caméra associée respective est à couleurs et l'ordinateur exécute un programme de classification de couleurs qui associe aux projecteurs de lignes les traces lumineuses des projecteurs de lignes individuels dans l'image de la caméra couleur.

12. Appareil de détection selon l'une des revendications 7 à 10, **caractérisé en ce que** des capteurs de détection électronique de signaux engendrés par l'individu et susceptibles d'être évalués par l'ordinateur, et/ou des actionneurs capables d'engendrer des excitations physiques perceptibles par l'individu sont intégrés dans la plaque de support et/ou dans le dispositif de maintien.
